# EUROPÄISCHE PATENTSCHRIFT

(11) **EP 1 755 474 B1**
(45) Veröffentlichungstag und Bekanntmachung des Hinweises auf die Patenterteilung: **03.12.2008**
(21) Anmeldenummer: 04735613.4
(22) Anmeldetag: 01.06.2004
(51) Int. Cl.: A61B 17/80

(54) **OSTEOSYNTHESEPLATTE**
OSTEOSYNTHESIS PLATE
PLAQUE D'OSTEOSYNTHESE

(43) Veröffentlichungstag der Anmeldung: 28.02.2007
(73) Patentinhaber: Synthes GmbH, 4436 Oberdorf (CH)
(72) Erfinder: SCHWER, Stefan, 79540 Lörrach (DE); MARIÉTHOZ, Eric, CH-1997 Haute-Nendaz (CH); ANDERMATT, Daniel, CH-4313 Möhlin (CH); MARTINELLI, Orlando, CH-3380 Wangen an der Aare (CH)
(74) Vertreter: Rosenich, Paul
(86) Internationale Anmeldenummer: PCT/IB2004/001784
(87) Internationale Veröffentlichungsnummer: WO 2005/117732

(56) Entgegenhaltungen:
- EP-A2- 1 486 175
- WO-A2-98/51226
- US-A- 4 297 993
- US-A- 5 785 712
- HEARN, E.J.: "Mechanics of Materials, Volume 2 - The Mechanics of Elastic and Plastic Deformation of Solids and Structural Materials (3rd Edition)" 1997, ELSEVIER , AMSTERDAM NL , XP002315178 ISBN: 0-7506-3266-6 Gefunden im Internet: URL:http://www.knovel.com/knovel2/Toc.jsp? SpaceID=162&BookID=434&NodeID=846888380&Ac tion=Expand&Type=null&FilterMode=false#nod e846888380> Seite 410, Absatz 1 Seite 414, Absatz 2 Seite 429, Absatz 10.3.7.C - Seite 430 Seite 431, Absatz 10.3.7.E - Absatz 10.3.7.F Seite 434, Absatz 10.3.9. - Seite 435 Abbildungen 10.16,10.17,10.24,10.44,10.46
- PILKEY, WALTER D.: "Peterson's Stress Concentration Factors (2nd Edition)" 1997, JOHN WILEY & SONS , NEW YORK, USA , XP002315179 ISBN: 0-471-53849-3 Gefunden im Internet: URL:http://www.knovel.com/knovel2/Toc.jsp? BookID=583> Seite 225, Absatz 4 - Absatz 6 Seite 239, Zeile 8 - Zeile 9 Seite 240, Absatz 4.6.4. - Seite 241, Absatz 4.6.5. Abbildungen 4.30,4.79-4.84
- SYNTHES USA: "3.5mm LCP Proximal Humerus Plate" Dezember 2003 (2003-12), SYNTHES USA , U.S.A. , XP002315180 Gefunden im Internet: URL:http://products.synthes.com/prod_suppo rt/Product%20Support%20Materials/Technique %20Guides/SUSA/SUTG35LcpPrxhumPltJ4029C.pd f> Seiten 1,5,8,9

## Beschreibung

Die vorliegende Erfindung betrifft eine Osteosyntheseplatte gemäß dem Oberbegriff des Anspruchs 1.

Osteosyntheseplatten zur Implantation, anatomischen Reposition und inneren Schienung von Knochenfragmenten nach Frakturen sind in den verschiedensten Formen und Ausgestaltungen weithin bekannt. Der Erfolg einer Frakturversorgung wird wesentlich von der Stabilität der Implantate bestimmt. Um eine Heilung der Fraktur vor dem Versagen des Implantates sicherzustellen, ist die Stabilität kritisch. Sie soll möglichst hoch sein, ohne dass das Implantat allzu dick und somit zu rigide wird. Eine zu hohe Dicke des Implantates könnte zu einer Störung der Weichteile führen. Ferner fehlt einem Implantat, das auf Grund einer zu hohen Dicke vergleichsweise steif ist, die für die Heilung notwendige Elastizität.

Osteosyntheseplatten weisen gewöhnlich eine Reihe an Bohrlöchern auf, durch die Knochenschrauben für das Fixieren der Implantate am Knochen und damit für die Fixierung der Fraktur hindurchgeführt werden. Vor allem bei Implantaten, die im artikluären Bereich eingesetzt werden, sind häufig mehrere Bohrlöcher vergleichsweise eng benachbart zueinander angeordnet. Dies führt vor allem bei konvexen Platten zu einer Reduktion der Stabilität in diesem Bereich. Dort ist der tragende Querschnitt auf Grund der Bohrungen geschwächt. Der zwischen den beiden Bohrlöchern noch verbleibende Bereich ist zum einen isoliert und liegt zum anderen auf Grund der konvexen Ausformung vergleichsweise weit von der neutralen Faser der Platte entfernt. Infolgedessen kommt es bei Biegebelastungen an der konvexen Seite zu einer Spannungsüberhöhung, die zum Versagen der Platte gerade in diesem Bereich führen könnte. Kritisch für die Stabilität einer Osteosyntheseplatte insgesamt ist somit ein vergleichsweise kleiner Bereich.

Dieser entscheidet unter Umständen über Erfolg oder Misserfolg einer Frakturversorgung.

Aufgabe der vorliegenden Erfindung ist es daher, die Stabilität von Osteosyntheseplatten zu verbessern. Insbesondere soll die Stabilität von Osteosyntheseplatten dort verbessert werden, wo zumindest zwei Bohrlöcher vergleichsweise nahe beieinander liegen.

Diese und andere Aufgaben werden erfindungsgemäß durch eine Osteosyntheseplatte nach Anspruch 1 gelöst. Vorteilhafte Ausgestaltungen sind in den abhängigen Ansprüchen zu finden.

Eine Osteosyntheseplatte gemäß der Erfindung weist zumindest zwei benachbart angeordnete Bohrlöcher auf. Erfindungsgemäß weist die Platte die Merkmale des Anspruchs 1 auf. Wie oben ausgeführt, treten bei benachbart angeordneten Bohrlöchern bei Biegebelastungen an der konvexen Seite Spannungsüberhöhungen auf. Auf den ersten Blick scheint es daher nicht sehr vorteilhaft zu sein, an einer Stelle der höchsten Belastung noch weiteres Material zu entfernen. Überraschenderweise ist jedoch das Gegenteil der Fall. Durch die zumindest eine Kerbe wird erreicht, dass Spannungsspitzen abgebaut werden und die maximale Biegebelastung auf einen breiteren Steg verteilt wird. Ferner wird ein Teil der Last auf die Außenseite der Platte geleitet. Der Bereich an der Außenseite der Bohrlöcher ist gewöhnlich wesentlich breiter als der zwischen den Bohrlöchern verbleibende Steg. Infolgedessen kann dieser auch mehr Last aufnehmen. Eine solche Entlastungskerbe ist vor allem im Rahmen einer winkelstabilen Plattenosteosynthese vorteilhaft, da bei der winkelstabilen Verankerung die gesamte Last von der Platte getragen wird.

Im Stand der Technik sind bereits Platten bekannt, die an gewissen Stellen Einschnitte bzw. Einkerbungen aufweisen. Eine Osteosyntheseplatte wie im Oberbegriff des Anspruch 1 ist aus US 5 785 712 bekannt. Terner sind Unterschnitte bekannt. Hierbei wird die Osteosyntheseplatte an der Unterseite mit Kerben versehen, wodurch die Kontaktfläche zwischen Platte und Knochen reduziert wird. Dadurch wird die periostale Blutversorgung weniger gestört, was die Heilung verbessert. Ferner sind seitliche Einschnitte bei Rekonstruktionsplatten bekannt, wo sie für eine bessere Biegbarkeit der Platte senkrecht zur Längsrichtung sorgen. Ohne diese Einschnitte besteht das Risiko, dass sich die Platte nur über die Materialschwächung durch das Bohrloch verbiegt, wodurch sich die Form des Bohrloches verändert, was bei einer Fixation mittels einer Knochenschraube nachteilig sein kann. Wichtig ist jedoch, dass sämtliche dieser Einkerbungen und Einschnitte gerade nicht im unmittelbaren Bereich bzw. der unmittelbaren Umgebung von Bohrlöchern angebracht werden. Die Querschnitte im Bereich der Bohrlöcher würden durch oben genannte Einschnitte geschwächt werden. Außerdem lässt sich nur durch einen entsprechend höheren Abstand der Einschnitte von den Bohrlöchern das Verziehen der Bohrlöcher durch das Verbiegen der Platte verhindern. Infolgedessen können die bekannten Osteosyntheseplatten, ob mit oder ohne bekannte Einschnitte, zur Lösung der Aufgabe nicht beitragen. Keine der bekannten Einschnitte und Einkerbungen führt zu einer Verteilung oder Ablenkung der Last weg von dem kritischen Bereich zwischen den Bohrlöchern.

Die Bezugszeichenliste und die Zeichnung sind zusammen mit den in den Ansprüchen beschriebenen, beziehungsweise geschützten Gegenständen integrierender Bestandteil der Offenbarung dieser Anmeldung.

### Figurenbeschreibung

Die Erfindung kann noch besser verstanden werden, indem auf die beiliegenden beispielhaften Figuren verwiesen wird. Sie werden zusammenhängend und übergreifend beschrieben. Gleiche Bezugszeichen bedeuten gleiche Bauteile, Indices geben funktionengleiche Bauteile an.

Es zeigen dabei:
Fig. 1 einen Ausschnitt aus einer nicht erfindungsgemäßen generischen Platte in Seitenansicht (A), Aufsicht (B) und perspektivischer Ansicht (C); und
Fig. 2 ein Ausführungsbeispiel einer erfindungsgemäßen Osteosyntheseplatte in Seitenansicht (A), Aufsicht (B) und perspektivischer Darstellung (C).

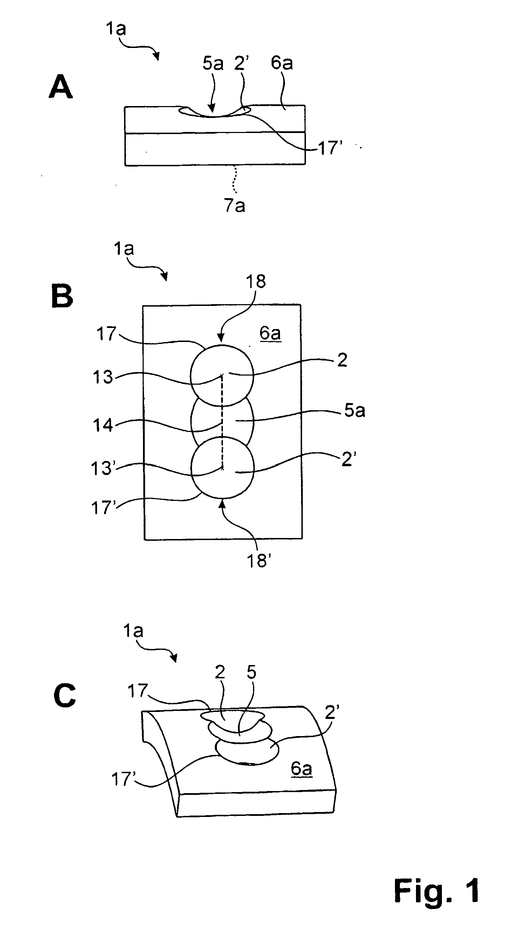

Fig. 1 zeigt einen Ausschnitt aus einer generischen Platte 1a in verschiedenen Ansichten. Bei der generischen Platte handelt es sich um ein Modell für eine Osteosyntheseplatte. Der in Fig. 1 gezeigt Ausschnitt der generischen Platte 1a weist zwei Bohrlöcher 2, 2' auf. Die beiden Bohrlöcher 2. 2' durchdringen die Platte, so dass eine Schraube eingeführt werden kann. Zwischen den Bohrlöchern 2, 2' ist eine Kerbe 5a angeordnet. Wie sich aus der Zeichnung der Fig. 1B ergibt, befindet sich diese Kerbe 5a zwischen den beiden Bohrlöchern 2, 2'. Anders ausgedrückt, verbindet die Kerbe 5a die beiden Bohrlöcher 2, 2'; sie verläuft von Bohrloch 2 zu Bohrloch 2'. Die generische Platte 1a ist eine gewölbte Platte. Sie weist eine konvexe Seite 6a und eine konkave Seite 7a auf. Wie insbesondere aus der Fig. 1C ersichtlich ist, ist die Kerbe 5a auf der konvexen Seite 6a angeordnet. Die konkave Seite 7a bleibt unverändert.

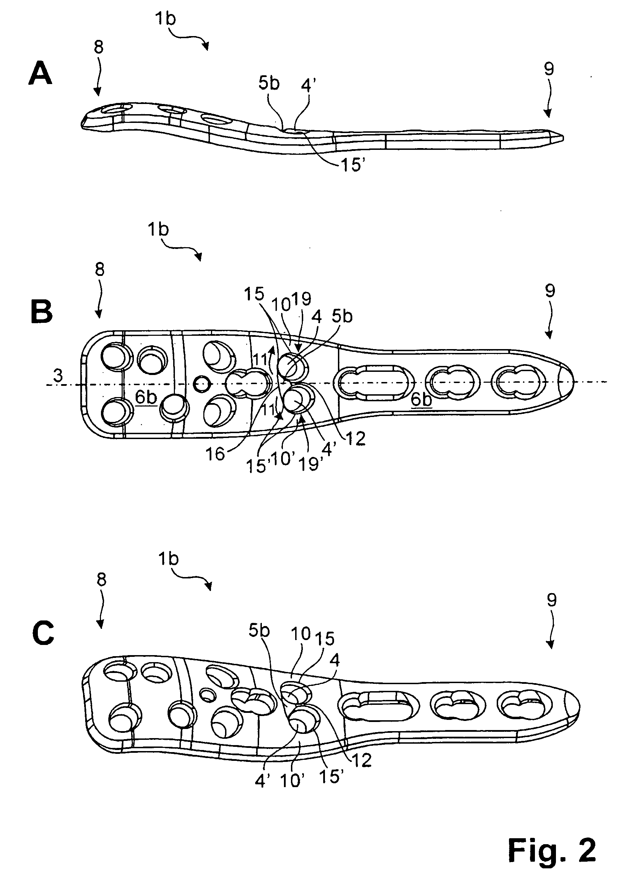

Fig. 2 zeigt eine Osteosyntheseplatte 1b in verschiedenen Ansichten. Die Osteosyntheseplatte 1b weist verschiedenartige Bohrlöcher auf. Unter dem Begriff "Bohrloch" wird hierin jede Öffnung oder Bohrung in der erfindungsgemäßen Platte verstanden, durch die Mittel für die Befestigung der Platte mit und an einem Knochen eingeführt werden können. So sind damit beispielsweise sowohl zylindrische als auch konische Bohrlöcher, ebenso wie Langlöcher und Bohrlöcher mit Gewinde, sowie deren Kombinationen umfasst. Wie aus Fig. 2 ersichtlich ist, sind die beiden Bohrlöcher 4, 4' im mittleren Bereich der Osteosyntheseplatte 1b vergleichsweise eng benachbart angeordnet. Im Bereich der beiden Bohrlöcher 4, 4' kommt es bei Biegebelastung an der konvexen Seite 6b zu einer Spannungsüberhöhung, wie oben ausgeführt. Gemäss der Erfindung erstreckt sich zwischen diesen Bohrlöchern 4, 4' eine Kerbe 5b. Durch das Anbringen der Kerbe 5b wird die Last, die auf dem Steg 12 im Bereich zwischen den beiden Bohrlöchern 4, 4' ruht, gleichmäßiger verteilt, z. B. auf die seitlichen Stege 10, 10' abgelenkt, wie angedeutet durch die Pfeile 11. Insbesondere aus der Darstellung der Fig. 2B wird deutlich, dass die seitlichen Stege 10, 10' breiter sind als der mittlere Steg 12. Infolgedessen kann der Steg 10, 10' auch mehr Last aufnehmen als der Steg 12. Überdies wird durch das Anbringen der Kerbe 5b das Flächenträgheitsmoment nur geringfügig verringert, wobei sich gleichzeitig der Randfaserabstand überproportional verkürzt. Dies trägt wesentlich zur Erhöhung der Stabilität der Platte bei.

Aus der Fig. 2 ist ersichtlich, dass das proximale Ende 8, im Gegensatz zum distalen Ende 9, löffelartig geformt, das heißt gewölbt, ist. Dadurch ist die Osteosyntheseplatte 1b sowohl für den linken als auch für den rechten proximalen Humerus einsetzbar. Durch diese gewölbte, löffelartige Gestaltung treten aber gerade diese Biegebelastungen, wie oben besprochen, auf. Deshalb ist die Entlastungskerbe vor allem bei derartigen Osteosyntheseplatten äußerst nützlich.

Bei der Kerbe 5 handelt es sich um eine Ausnehmung, die in die Osteosyntheseplatte eingebracht ist. Anders kann sie auch mit den Begriffen "Einschnitt" oder "Nute" beschrieben werden. Die Kerbe 5 ist insbesondere gerundet, um den Einfluss der Kerbwirkung zu reduzieren.

Die Tiefe der Kerbe 5 hängt von der Krümmung der Osteosyntheseplatte 1 ab. Je stärker diese gekrümmt ist, desto tiefer kann die Kerbe ausgebildet sein. Als eine Hilfe zur Bestimmung der bevorzugten Tiefe der Kerbe 5 kann die Seitenansicht einer Platte dienen. Wenn in Seitenansicht, wie in Fig. 2 A dargestellt, der zwischen den Bohrlöchern 4, 4' vorhandene Steg 12 nicht mehr über den Rand der Bohrlöcher hinaus steht, dann weist die Kerbe 5 die bevorzugte Tiefe auf. Die Krümmung der Platte soll die Kerbe in Seitenansicht verdecken.

Anders ausgedrückt weist die Kerbe 5 eine Tiefe auf, welche im Wesentlichen der Tiefe der Kante 15, 15' bzw. 17, 17' des Bohrloches 4, 4' bzw. 2, 2' entspricht, die sich auf dem Bereich 19, 19' bzw. 18, 18' des Bohrloches 4, 4' bzw. 2, 2' befindet, der quer zur Längsachse der Kerbe 5 verläuft und der weiter von der Kerbe entfernt liegt. Die Kerbe ist folglich bevorzugt in etwas so tief wie die gegenüberliegende Kante des Bohrloches tief liegt, was insbesondere aus der Fig. 1A und der Fig. 2A deutlich hervorgeht. Unter der "Tiefe" der Bohrlochkante wird dabei der Abstand zwischen der Kante des Bohrloches und der Ebene, die auf der Oberseite der Osteosyntheseplatte liegt, verstanden. Aus dieser Definition wird auch deutlich, dass die Tiefe von der Krümmung der Platte, dem Abstand der Bohrlöcher, zwischen denen die Kerbe angebracht wird, und der Dicke der Platte abhängt. Die Tiefe kann jedoch variieren. Wichtig ist allerdings, dass die Kerbe nur so tief ist, dass keine neue exponierte Zone beispielsweise an der Außenseite der Bohrlöcher (in Fig. 2 im Bereich des Bezugszeichens10, 10') erzeugt wird.

Wie oben ausgeführt, wird die Kerbe 5 zwischen zwei benachbart angeordneten Bohrlöchern angebracht. Ein Ausführungsbeispiel sieht vor, dass sich die Kerbe 5 koaxial zu einer Geraden 14 erstreckt, die die beiden Mittelpunkte 13, 13' der Bohrlöcher 2, 2' verbindet, wie dargestellt in Fig. 1B. Die Längsachse der Kerbe 1a fällt folglich mit der Geraden 14 zusammen. Bei Plattensonderformen, insbesondere gekrümmten Osteosyntheseplatten, kann die Kerbe 5 aus der Mitte des Steges verschoben sein. So ist vorgesehen, dass die Entlastungskerbe zu dieser Verbindung der Mittelpunkte parallel verschoben ist, wie beispielsweise in Fig. 2B dargestellt. Hier befindet sich die Kerbe 5b in Richtung zum proximalen Ende 8 der Osteosyntheseplatte 1b hin verschoben. Die Kerbe 5b bildet die Tangente der beiden Bohrlöcher 4, 4'. Vorteilig ist, wenn sich der proximale Bereich der Kante 16 der Kerbe 5 in etwa auf der Höhe oder darüber befindet, auf der sich der proximale Bereich der Kante 15, 15' des Bohrloches 4, 4' befindet. Dadurch wird die Lastverteilung gewährleistet.

Wie sich insbesondere aus der Fig. 2 ergibt, ist die Kerbe 5 quer zur Längsachse 3 der Osteosyntheseplatte 1b angeordnet.

Die Bohrlöcher, zwischen denen die Kerbe angeordnet ist, sind benachbart angeordnet. Unter "benachbart" wird hierin verstanden, dass die Bohrlöcher vergleichsweise nahe beieinander liegen. Das genaue Maß des Abstandes zwischen den benachbarten Bohrlöchern, bei der die vorliegende Erfindung ihre vorteilhaften Wirkungen entfaltet, hängt wiederum von der Krümmung der Osteosyntheseplatte ab. Überdies ist die Größe des Implantates entscheidend. Ferner hängt sie zu einem gewissen Grad auch von der Gesamtanzahl der auf dem Implantat angeordneten Bohrlöcher ab. In jedem Fall sind zwei Bohrlöcher im Sinne der Erfindung benachbart angeordnet, wenn zwischen diesen nur ein schmaler Steg verbleibt, der zu Spannungsüberhöhungen an dieser Stelle führt.

Die beiden Bohrlöcher 2, 2' bzw. 4, 4' können noch als benachbart im Sinne der Erfindung angesehen werden, wenn ihr Abstand nicht so groß ist, dass die oben definierte Tiefe der Kerbe 5 die Dicke der Osteosyntheseplatte 1 übertrifft. Dies bedeutet, dass der Abstand der benachbart angeordneten Bohrlöcher 2, 2' bzw. 4, 4' von der Tiefe der Kerbe 5 abhängt, dahingehend, dass die Kerbe nicht tiefer ist als die Osteosyntheseplatte 1 dick ist. Wenn die Tiefe der Kerbe 5 also an die Tiefe der gegenüberliegenden Kante 15, 15' bzw. 17, 17' angepasst wird, so wird bei einer gekrümmten Osteosyntheseplatte 1 irgendwann ein Punkt erreicht, bei dem sich diese Kante 15, 15' bzw. 17, 17' unterhalb einer durch die Unterseite der Platte aufgespannten Ebene befindet, sprich tiefer liegt als die Unterseite der Platte. Wäre die Kerbe 5 dann immer noch so tief wie die Kante 15, 15', 17, 17', so wäre diese Tiefe größer als die Dicke der Platte und letztere folglich durchbrochen. Hieraus wird auch deutlich, dass der Abstand der Bohrlöcher bei stärker gekrümmten Platten geringer sein wird als bei weniger stark gekrümmten. Hier können die Bohrlöcher, zwischen denen die Kerbe gemäß der Erfindung angebracht wird, weiter auseinander liegen und die Kerbe kann trotzdem ihre erfindungsgemäße Funktion erfüllen.

### Bezugszeichenliste

- 1 -: Osteosyntheseplatte
- 2 -: Bohrloch
- 3-: Längsachse
- 4 -: Bohrloch
- 5 -: Kerbe
- 6 -: konvexe Seite
- 7 -: konkave Seite
- 8 -: proximales Ende
- 9 -: distales Ende
- 10 -: seitlicher Steg
- 11 -: Pfeil
- 12 -: Steg
- 13 -: Mittelpunkt
- 14 -: Gerade
- 15 -: Kante
- 16 -: Kante
- 17: Kante
- 18: Bereich
- 19: Bereich

## Patentansprüche

1. Osteosyntheseplatte (1) mit zumindest zwei benachbart angeordneten Bohrlöchern (4, 4') wobei auf der konvexen Seite (6) der Osteosyntheseplatte (1) zwischen den zumindest zwei benachbart angeordneten Bohrlöchern mindestens eine Kerbe (5) angeordnet ist, wobei
**dadurch gekennzeichnet, dass**
die Längsachse der Kerbe (5) eine Tangente zu den beiden Bohrlöchern (4, 4') bildet, und wobei zwischen den Bohrlöchern (4, 4') ein schmaler Steg (12) besteht, der schmäler ist als die seitlichen Stege (10, 10') an der Außenseite der Bohrlöcher (4, 4').

2. Osteosyntheseplatte nach Anspruch 1 **dadurch gekennzeichnet, dass** sich der proximale Bereich der Kante (16) der Kerbe (5) im Wesentlichen auf der Höhe oder darüber befindet, auf der sich der proximale Bereich der Kante (15) der zumindest zwei Bohrlöcher (4, 4') befindet, zwischen denen die Kerbe (5) angeordnet ist.

3. Osteosyntheseplatte nach Anspruch 1 oder 2 **dadurch gekennzeichnet, dass** die Kerbe (5) gerundet ist.

4. Osteosyntheseplatte nach einem der vorhergehenden Ansprüche, **dadurch gekennzeichnet, dass** die Kerbe (5) eine Tiefe aufweist, welche im Wesentlichen der Tiefe der Kante (15, 17) des Bohrloches entspricht, die sich auf dem Bereich (18, 19) des Bohrloches (4, 4') befindet, der quer zur Längsachse der Kerbe verläuft und der weiter von der Kerbe entfernt liegt.

5. Osteosyntheseplatte nach Anspruch 4, **dadurch gekennzeichnet, dass** der Abstand der benachbart angeordneten Bohrlöcher (4, 4') von der Tiefe der Kerbe (5) abhängt, dahingehend, dass die Kerbe nicht tiefer ist als die Osteosyntheseplatte (1) dick ist.

6. Osteosyntheseplatte nach einem der vorhergehenden Ansprüche, **dadurch gekennzeichnet, dass** die Kerbe (5) quer zur Längsachse (3) der Osleosyntheseplatte (1) angeordnet ist.

7. Osteosyntheseplatte nach einem der vorhergehenden Ansprüche, **dadurch gekennzeichnet, dass** der proximale Teil der Osteosyntheseplatte löffelartig gewölbt ist, so dass die Osteosyntheseplatte sowohl für den linken als auch den rechten proximalen Humerus einsetzbar ist.

## Claims

1. An osteosynthesis plate (1) comprising at least two drilled holes (4, 4') arranged adjacent to one another, wherein at least one notch (5) is arranged on the convex side (6) of the osteosynthesis plate (1) between the at least two drilled holes arranged adjacent to one another, **characterized in that** the longitudinal axis of the notch (5) makes a tangent to the two drilled holes (4, 4') and wherein between the drilled holes (4, 4') a narrow web (12) exists, which is narrower than the lateral webs (10, 10') on the outer side of the drilled holes (4, 4').

2. The osteosynthesis plate according to Claim 1, **characterized in that** the proximal region of the edge (16) of the notch (5) is present substantially at the height at which the proximal region of the edge (15) of the at least two drilled holes (4, 4') is present, between which the notch (5) is arranged, or above said height.

3. The osteosynthesis plate according to Claim 1 or 2, **characterized in that** the notch (5) is rounded.

4. The osteosynthesis plate according to any of the preceding claims, **characterized in that** the notch (5) has a depth which substantially corresponds to the depth of the edge (15, 17) of the drilled hole which is present on the region (18, 19) of the drilled hole (4, 4') which runs transversely to the longitudinal axis of the notch and which lies further away from the notch.

5. The osteosynthesis plate according to Claim 4, **characterized in that** the spacing of the drilled holes (4, 4') arranged adjacent to one another depends on the depth of the notch (5) **in that** the notch is not deeper than the thickness of the osteosynthesis plate (1).

6. The osteosynthesis plate according to any of the preceding claims, **characterized in that** the notch (5) is arranged transversely to the longitudinal axis (3) of the osteosynthesis plate (1).

7. The osteosynthesis plate according to any of the preceding claims, **characterized in that** the proximal part of the osteosynthesis plate is arched in a spoon-like manner so that the osteosynthesis plate can be used both for the left and for the right proximal humerus.

## Revendications

1. Plaque d'ostéosynthèse (1) comportant au moins deux perforations disposées à proximité l'une de l'autre (4, 4'), au moins une encoche (5) étant disposée sur le côté convexe (6) de la plaque d'ostéosynthèse (1) entre les au moins deux perforations disposées à proximité l'une de l'autre, **caractérisée en ce que** l'axe longitudinal de l'encoche (5) forme une tangente par rapport aux deux perforations (4, 4') et sachant qu'il y a entre les perforations (4, 4') une étroite traverse (12) qui est plus étroite que les traverses latérales (10, 10') au niveau de la face extérieure des perforations (4, 4').

2. Plaque d'ostéosynthèse selon la revendication 1, **caractérisée en ce que** la zone proximale de l'arête (16) de l'encoche (5) se trouve sensiblement à la hauteur ou au dessus de la hauteur où se trouve la zone proximale de l'arête (15) des aux moins deux perforations (4, 4') entre lesquelles l'encoche (5) est disposée.

3. Plaque d'ostéosynthèse selon la revendication 1 ou 2, **caractérisée en ce que** l'encoche (5) est arrondie.

4. Plaque d'ostéosynthèse selon une des revendications précédentes, **caractérisée en ce que** l'encoche (5) a une profondeur qui équivaut sensiblement à la profondeur de l'arête (15, 17) de la perforation et qui se trouve au niveau (18, 19) de la perforation (4, 4') qui s'étend transversalement par rapport à l'axe longitudinal de l'encoche et qui se situe plus loin de l'encoche.

5. Plaque d'ostéosynthèse selon la revendication 4, **caractérisée en ce que** la distance entre les perforations (4, 4') disposées à proximité l'une de l'autre dépend de la profondeur de l'encoche (5) du fait que l'encoche n'est pas plus profonde que la plaque d'ostéosynthèse (1) n'est épaisse.

6. Plaque d'ostéosynthèse selon une des revendications précédentes, **caractérisée en ce que** l'encoche (5) est disposée transversalement par rapport à l'axe longitudinal (3) de la plaque d'ostéosynthèse (1).

7. Plaque d'ostéosynthèse selon une des revendications précédentes, **caractérisée en ce que** la partie proximale de la plaque d'ostéosynthèse est bombée à la manière d'une cuillère, de sorte que la plaque d'ostéosynthèse est utilisable pour l'humérus proximal gauche aussi bien que droit.
